# EUROPEAN PATENT APPLICATION

(11) **EP 4 327 772 A1**
(43) Date of publication of application: **28.02.2024**
(21) Application number: 22792031.1
(22) Date of filing: 20.04.2022
(51) Int. Cl.: A61C 9/00, G06T 1/00, A61B 5/00, G16H 30/00, A61B 18/20

(54) **INTRAORAL IMAGE PROCESSING DEVICE AND INTRAORAL IMAGE PROCESSING METHOD**

(30) Priority: 22.04.2021 KR 20210052364
(71) Applicant: Medit Corp., Seoul 07207 (KR)
(72) Inventor: LEE, Dong Hoon, Seoul 07207 (KR); KIM, Dusu, Seoul 07207 (KR)
(74) Representative: Taor, Simon Edward William
(86) International application number: PCT/KR2022/005665
(87) International publication number: WO 2022/225332

(57) **Abstract**

Disclosed is an intra image processing method including obtaining a first intraoral image, and obtaining a second intraoral image, into which input additional information is embedded, by embedding the input additional information into at least a partial area of the first intraoral image.

## Description

### Technical Field

The disclosed embodiments relate to an intraoral image processing device and an intraoral image processing method, and more particularly, to a device and method for processing an intraoral image.

### Background Art

Recently, for dental treatment of patients, intraoral scanners that are inserted into oral cavities of patients and obtain intraoral images have been used. Intraoral scanners may obtain two-dimensional image data by scanning oral cavities of patients. The two-dimensional image data may be image data representing the scanned oral cavities. In addition, a computing device, such as a personal computer (PC) connected to an intraoral scanner, may generate a three-dimensional intraoral model by using two-dimensional image data obtained by an intraoral scanner. Hereinafter, a computing device that processes two-dimensional scan data is referred to as an intraoral image processing device.

A three-dimensional intraoral model is delivered to a dental laboratory, and the dental laboratory uses a three-dimensional intraoral model to produce prosthetics that fit a patient's intraoral cavity. Specifically, users such as dental technicians may produce artificial structures, such as prosthetics, by using a received three-dimensional intraoral model.

There is a need to provide an intraoral image processing method and an intraoral image processing device so that users such as dental technicians may more conveniently and accurately produce artificial structures for use in dental treatment.

### Disclosure

### Technical Solution

An intra image processing method according to an embodiment may include obtaining a first intraoral image and obtaining a second intraoral image, into which input additional information is embedded, by embedding the input additional information into at least a partial area of the first intraoral image.

### Description of Drawings

FIG. 1 is a diagram for describing an intraoral image processing system according to an embodiment.
FIG. 2 is a diagram for describing a method, performed by an intraoral scanner, of obtaining surface data, according to an embodiment.
FIG. 3 is a block diagram of an intraoral image processing device according to an embodiment.
FIG. 4 is an internal block diagram of an intraoral image processing device according to an embodiment.
FIG. 5 is a diagram illustrating an obtained second intraoral image, according to an embodiment.
FIG. 6 is a diagram illustrating a user interface screen including an additional information input area, according to an embodiment.
FIG. 7 is a diagram for describing a method of embedding additional information into an intraoral image, according to an embodiment.
FIG. 8 is a diagram for describing a method of embedding additional information into an intraoral image, according to another embodiment.
FIG. 9 is a diagram for describing embedding additional information into scan data by changing a color of a mesh, according to an embodiment.
FIG. 10 is a flowchart illustrating a method of embedding additional information into an intraoral image, according to an embodiment.

### Mode for Invention

In an embodiment, the method may further include identifying an additional information input area for displaying the additional information in the first intraoral image.

In an embodiment, the identifying of the additional information input area may include identifying the additional information input area in remaining areas other than an oral cavity area of the first intraoral image.

In an embodiment, the method may further include receiving a selection of at least one of a tooth and gingiva as a target, wherein the identifying of the additional information input area may include identifying the additional information input area in remaining area other than an area within a certain range from the at least one selected from the tooth and the gingiva.

In an embodiment, the identifying of the additional information input area may include identifying the additional information input area from the first intraoral image by using a neural network trained to identify additional information input areas from a plurality of intraoral images.

In an embodiment, the method may further include outputting a user interface screen for selecting the additional information input area, wherein the identifying of the additional information input area may include identifying a selected area as the additional information input area in response to the user interface screen.

In an embodiment, the method may further include outputting at least one of a user interface screen on which the identified additional information input area is displayed and a user interface screen on which the input additional information is displayed in the identified additional information input area.

In an embodiment, when the input additional information is greater than or equal to a certain size and when there are a plurality of additional information input areas where the additional information is displayed, the outputting of the user interface screen on which the input additional information is displayed may include outputting an identifier indicating a position of the additional information input area instead of the additional information, or outputting the additional information input area whose size is reduced.

In an embodiment, the additional information may include at least one of a text and an image.

In an embodiment, the first intraoral image may be three-dimensional scan data, and the obtaining of the second intraoral image into which the additional information is embedded may include obtaining the second intraoral image by replacing variables or color values of pixels of a two-dimensional image mapped to the first intraoral image with values corresponding to the additional information and embedding the additional information into the first intraoral image.

In an embodiment, the first intraoral image may be three-dimensional scan data expressed as at least one of dots and mesh, and the obtaining of the second intraoral image into which the additional information is embedded may include obtaining the second intraoral image by changing a color of at least one of a point, a vertex and a polygon including the vertex in the first intraoral image located at a position corresponding to an outline of at least one of a text and an image included in the additional information, and embedding the additional information in the first intraoral image.

An intraoral image processing device according to an embodiment may include a processor configured to execute one or more instructions, wherein the processor is configured to execute the one or more instructions to: obtain a first intraoral image; and obtain a second intraoral image, into which input additional information is embedded, by embedding the input additional information into at least a partial area of the first intraoral image.

The present disclosure clarifies the scope of the present application and explains the principles of the present application and discloses embodiments, so that those of ordinary skill in the art may carry out the present application. The disclosed embodiments may be implemented in various forms.

The same reference numerals refer to the same elements throughout the specification. The present disclosure does not explain all elements of the embodiments, and general descriptions in the technical field to which the present application belong or redundant descriptions between the embodiments are omitted. The term 'part' or 'portion' as used in the specification may be implemented in software or hardware. According to an embodiment, a plurality of 'parts' or 'portions' may be implemented as a single unit or element, or one 'part' or 'portion' may include a plurality of units or elements. Hereinafter, the operating principles and embodiments of the present disclosure are described with reference to the accompanying drawings.

In the present specification, an image may include an image representing at least one tooth or an oral cavity including at least one tooth (hereinafter referred to as an 'intraoral image').

In addition, in the present specification, an image may be a two-dimensional image of an object, or a three-dimensional intraoral model or a three-dimensional image representing an object three-dimensionally.

In addition, in the present specification, 'data' may refer to information required to represent an object two-dimensionally or three-dimensionally, for example, raw data obtained by using at least one camera.

Specifically, the raw data is data obtained so as to generate an intraoral image. The raw data may be data (e.g., two-dimensional data) obtained by at least one image sensor included in an intraoral scanner when a patient's oral cavity, which is an object, is scanned by using the intraoral scanner. The raw data obtained by the intraoral scanner may also be referred to as scan data or two-dimensional image data. The raw data may refer to two-dimensional images of different viewpoints obtained by a plurality of cameras when an object is scanned by using an intraoral scanner.

In the present specification, an 'object' is a subject to be scanned, and may include a human, an animal, or a part thereof. The object may include an oral cavity including at least one tooth. Together with or separately from this, the object may include a plaster model that imitates an oral cavity, a denture such as false teeth or artificial teeth, a dentiform having a tooth shape, and the like. For example, the object may include teeth, gingiva, at least a partial area of an oral cavity, and/or an artificial structure insertable into an oral cavity (e.g., orthodontic appliances, implants, crowns, inlays, onlays, orthodontic aids inserted into an oral cavity, etc.). The orthodontic appliances may include at least one of brackets, attachment, orthodontic screws, lingual orthodontic appliances, and removable retainers.

As described above, the three-dimensional intraoral model may be generated based on the two-dimensional scan data obtained by the intraoral scanner. The three-dimensional intraoral model may be transmitted to a dental laboratory, etc. so as to generate an artificial structure used in dental treatment.

Upon transmitting the three-dimensional intraoral model, the dentist may want to transmit additional information, such as special details or requests related to a patient's dental treatment, to the dental laboratory. To this end, the dentist may deliver a document containing the additional information to be transmitted to the dental laboratory, or may transmit an e-mail, a text message, or a file containing the additional information to the dental laboratory via a communication network.

However, because the document, the e-mail, the file, etc. are all generated and transmitted separately from the three-dimensional intraoral model, there is a possibility that incorrect information that does not fit the three-dimensional intraoral model may be transmitted. In addition, there is a problem that management is cumbersome in that the dental laboratory has to receive this additional information separately from the three-dimensional intraoral model and manage the additional information together with the three-dimensional intraoral model.

Therefore, in order to overcome the problems described above, the disclosed embodiments provide a method and device for transmitting additional information by including the additional information in an intraoral image such as a three-dimensional intraoral model.

Hereinafter, embodiments will be described in detail with reference to the drawings.

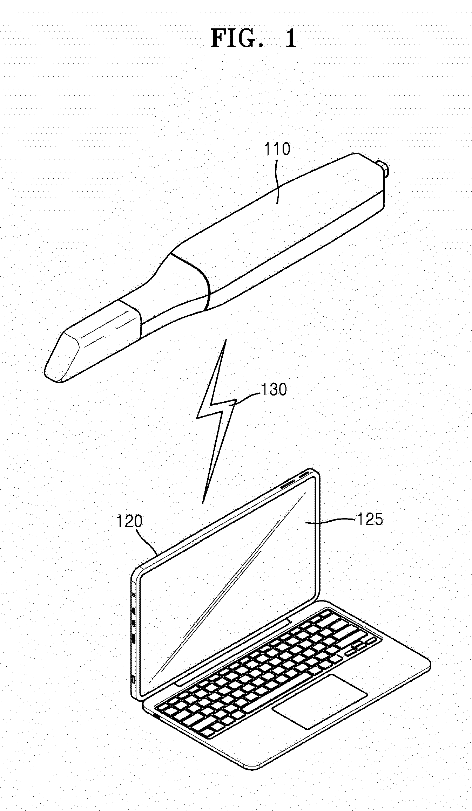

FIG. 1 is a diagram for describing an intraoral image processing system according to an embodiment.

Referring to FIG. 1, the intraoral image processing system may include a three-dimensional scanner 110 and an intraoral image processing device 120 connected to the three-dimensional scanner 110 via a communication network 130.

The three-dimensional scanner 110 is a medical device that obtains an image of an object. The object may include at least one of an oral cavity, a plaster model of the oral cavity, a denture, and a dentiform.

The three-dimensional scanner 110 may include at least one of a table scanner and an intraoral scanner. The table scanner may obtain scan data by scanning an object, for example, a plaster model, a denture, or a dentiform. The table scanner may obtain three-dimensional scan data of an object by scanning the object by using rotation of a table.

The intraoral scanner may be a handheld type scanner that scans the oral cavity while a user holds the scanner in his/her hand and moves the scanner. The three-dimensional scanner 110 may be inserted into the oral cavity and obtain an image of an oral cavity including at least one tooth by scanning teeth in a non-contact manner. In addition, the three-dimensional scanner 110 may have a shape to be inserted into and taken out from the oral cavity, and may scan a patient's oral cavity by using at least one image sensor (e.g., an optical camera, etc.). In order to image the surface of at least one of the object teeth, gingiva, and artificial structures insertable in the oral cavity (e.g., orthodontic appliances including brackets and wires, implants, artificial teeth, orthodontic aids to be inserted into the oral cavity, etc.), the three-dimensional scanner 110 may obtain surface information of an object as raw data.

Two-dimensional image data obtained by the three-dimensional scanner 110 may be transmitted to the intraoral image processing device 120 connected via the communication network 130.

The intraoral image processing device 120 may be any electronic device that is connected to the three-dimensional scanner 110 via a wired or wireless communication network 130 and may receive, from the three-dimensional scanner 110, two-dimensional image data obtained by scanning an object and generate, process, display, and/or transmit an intraoral image based on the received two-dimensional image data.

The intraoral image processing device 120 may generate at least one of the information generated by processing the two-dimensional image data and the intraoral image generated by processing the two-dimensional image data, based on the two-dimensional image data received from the three-dimensional scanner 110, and may display the generated information and the generated intraoral images on a display 125.

For example, the intraoral image processing device 120 may be a computing device, such as a smartphone, a laptop computer, a desktop computer, a personal digital assistant (PDA), and a tablet PC, but is not limited thereto.

The intraoral image processing device 120 may be present in the form of a server (or a server device) for processing an intraoral image.

The three-dimensional scanner 110 may transmit raw data obtained through intraoral scanning to the intraoral image processing device 120 as it is. In this case, the intraoral image processing device 120 may generate a three-dimensional intraoral image representing the oral cavity three-dimensionally, based on the received raw data. In addition, because the 'three-dimensional intraoral image' may be generated by modeling the internal structure of the oral cavity three-dimensionally, based on the received raw data, the three-dimensional intraoral image may also be referred to as a 'three-dimensional intraoral model.' Hereinafter, the model or the image representing the oral cavity two-dimensionally or three-dimensionally is collectively referred to as an 'intraoral image.'

The intraoral image processing device 120 may analyze and/or process the generated intraoral image and output or transmit the intraoral image to the display 125 and/or an external device.

As another example, the three-dimensional scanner 110 may obtain raw data through intraoral scanning, generate an image corresponding to the oral cavity, which is the object, by processing the obtained raw data, and transmit the image to the intraoral image processing device 120.

In an embodiment, the three-dimensional scanner 110 may obtain three-dimensional data representing the shape of the object by using the principle of triangulation based on pattern deformation by projecting pattern light onto the object and scanning the object onto which the pattern light is irradiated.

In an embodiment, the three-dimensional scanner 110 may obtain three-dimensional data regarding the object by using a confocal method. The confocal method is a non-destructive optical imaging technique for three-dimensional surface measurement, and may obtain an optical cross-sectional image with high spatial resolution by using a pinhole structure. The three-dimensional scanner 110 may obtain three-dimensional data by stacking two-dimensional images obtained along an axial direction.

However, this is only an example, and the three-dimensional scanner 110 may obtain an intraoral image from the raw data by using various methods in addition to the aforementioned method. The three-dimensional scanner 110 may transmit the three-dimensional data to the intraoral image processing device 120. In this case, the intraoral image processing device 120 may analyze, process, display, and/or transmit the received image.

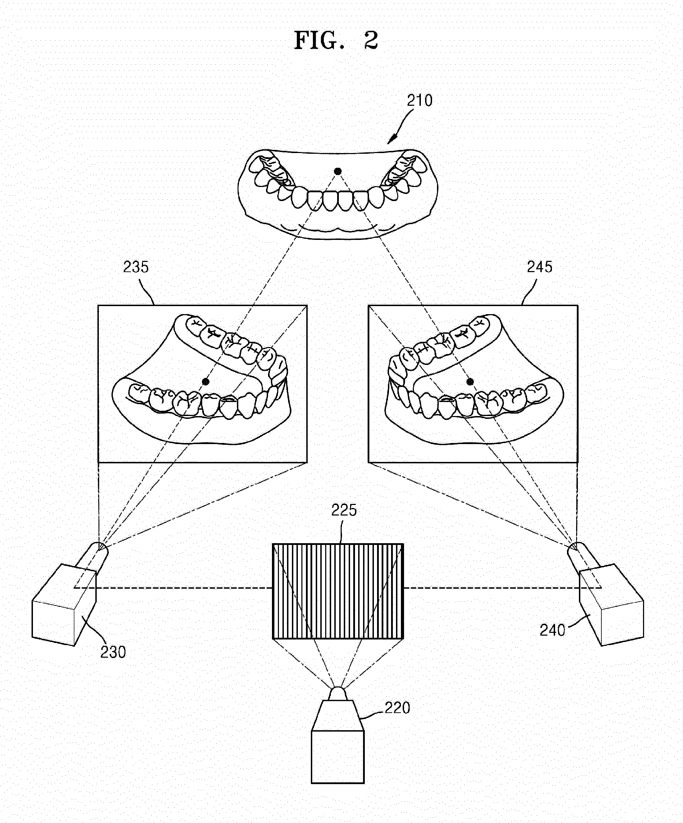

FIG. 2 is a diagram for describing a method, performed by the intraoral scanner, of obtaining surface data, according to an embodiment.

In an embodiment, the three-dimensional scanner 110 may obtain images by using at least one camera and obtain three-dimensional data based on the obtained images. In FIG. 2, the three-dimensional scanner 110 may be an optical three-dimensional scanner. The three-dimensional scanner 110 may use a 'structured light with stereo vision' method so as to obtain three-dimensional data regarding a surface of an object 210.

The three-dimensional scanner 110 may include two or more cameras 230 and 240 and a projector 220 capable of projecting structured light 225. In an embodiment, the three-dimensional scanner 110 may project the structured light 225 onto the object 210 and obtain an L image 235 corresponding to a left field of view and an R image 245 corresponding to a right field of view, respectively, from a first camera 230 corresponding to the left field of view and a second camera 240 corresponding to the right field of view. The L image 235 and the R image 245 may be reconstructed into a three-dimensional image frame representing the surface of the object 210.

The three-dimensional scanner 110 may continuously obtain a two-dimensional image frame including the L image 235 and the R image 245 of the object 210. The three-dimensional scanner 110 or the intraoral image processing device 120 may obtain a three-dimensional image frame representing the surface shape of the object 210 from the two-dimensional image frame including the L image 235 and the R image 245. In FIG. 2, a case where the three-dimensional scanner 110 obtains three-dimensional data from two images obtained by using the two cameras 230 and 240 has been described, but this is only one embodiment, and the three-dimensional scanner 110 may obtain an image by using only one of the two cameras 230 and 240.

The three-dimensional scanner 110 may obtain a plurality of two-dimensional frames by scanning the object 210 at regular time intervals (e.g., 10 frames to 30 frames per second) while moving around the object 210. The three-dimensional scanner 110 or the intraoral image processing device 120 may obtain a plurality of three-dimensional image frames from the two-dimensional image frames.

The intraoral image processing device 120 may obtain a three-dimensional intraoral model for the entire object 210 by merging or aligning the three-dimensional image frames.

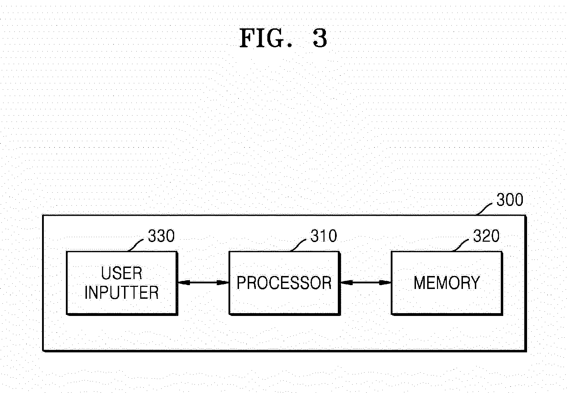

FIG. 3 is an internal block diagram of an intraoral image processing device according to an embodiment.

The intraoral image processing device 300 may also be referred to as a data processing device.

The intraoral image processing device 300 of FIG. 3 may be an example of the intraoral image processing device 120 of FIG. 1. Therefore, descriptions of parts redundant to those of the intraoral image processing device 120 of FIG. 1 are omitted.

The intraoral image processing device 300 may be an electronic device capable of generating, processing, displaying, and/or transmitting an intraoral image by using two-dimensional image data received from the three-dimensional scanner 110.

Referring to FIG. 3, the intraoral image processing device 300 may include a processor 310, a memory 320, and a user inputter 330.

Specifically, the intraoral image processing device 300 includes the user inputter 330, the memory 320 that stores one or more instructions, and the processor 310 that executes the one or more instructions stored in the memory. The processor 310 executes the one or more instructions to obtain a first intraoral image and obtain a second intraoral image, into which additional information is embedded, by embedding the additional information input through the user inputter into at least a partial area of the first intraoral image.

For example, the memory 320 according to an embodiment may store at least one instruction. The memory 320 may store the at least one instruction or program to be executed by the processor 310.

In addition, the memory 320 may store data received from the three-dimensional scanner 110, for example, raw data obtained through intraoral scanning. Alternatively, the memory 320 may store an intraoral image representing an oral cavity three-dimensionally.

In an embodiment, the memory 320 may include one or more instructions for identifying an additional information input area in the intraoral image.

In an embodiment, when the processor 310 identifies the additional information input area by using a neural network, the memory 320 may store the neural network.

In an embodiment, the memory 320 may store dedicated software for inputting additional information. The dedicated software for inputting additional information may be referred to as a dedicated program, a dedicated tool, or a dedicated application.

In an embodiment, the memory 320 may include one or more instructions for embedding additional information into the intraoral image.

The user inputter 330 according to an embodiment may receive a user input for controlling the intraoral image processing device 300. The user inputter 330 may include a user input device including a touch panel that detects a user touch, a button that receives a push operation from a user, or a mouse or a keyboard that points or selects a point on a user interface screen, but is not limited thereto. In addition, the user inputter 330 may include a voice recognition device for voice recognition. For example, the voice recognition device may be a microphone, and the voice recognition device may receive a user's voice command or voice request. Accordingly, the processor 310 may perform control so that an operation corresponding to the voice command or the voice request is performed.

In an embodiment, the user inputter 330 may receive additional information input from a user such as a dentist. The additional information may refer to information that the user wants to record additionally to the intraoral image. For example, the additional information may include information about the oral cavity, information about the patient, and other requests. As another example, the additional information may include information used or referenced when an artificial structure is manufactured based on the intraoral image.

The dentist may use the user inputter 330 to input, as the additional information, comments he or she wants to make regarding teeth or gingiva included in three-dimensional scan data. For example, the user (e.g., the dentist) may type additional information in text by using a keyboard, etc., or generate an image as additional information by using a mouse or a touch panel, and may input the typed additional information or the generated image as additional information.

The addition information may include at least one of symbols, texts, images, and colors. The texts included in the additional information may include at least one of language symbols, such as Hangul, alphabets, and Chinese characters, special characters, numbers, and punctuation marks. In addition, the additional information may include at least one of figures, arrows, lines, patterns, and symbols. For example, the additional information may be a combination of texts and symbols representing information about the patient. In addition, the additional information may be expressed two-dimensionally or three-dimensionally. For example, the additional information may be a combination of texts and symbols representing information about the patient.

The user may input additional information by using a program, a tool, or an application for inputting additional information, and may edit the additional information.

The processor 310 according to an embodiment may control overall operations of the intraoral image processing device 300. The processor 310 may execute at least one instruction to control an intended operation to be performed. The at least one instruction may be stored in the memory 320 included in the intraoral image processing device 300 or an internal memory (not shown) included in the processor 310, separately from the processor 310.

Specifically, the processor 310 may execute the at least one instruction to control at least one element included in the intraoral image processing device 300 so that an intended operation is performed. Therefore, even when a case where the processor 310 performs certain operations is described as an example, it may mean that the processor 310 controls at least one element included in the intraoral image processing device 300 so that the certain operations are performed.

In an embodiment, the processor 310 may obtain a first intraoral image. The first intraoral image may be an intraoral image generated based on data obtained by scanning an object. The first intraoral image may be a three-dimensional scan model. The three-dimensional scan model may also be referred to as three-dimensional scan data. The first intraoral image may be an image obtained by directly scanning the oral cavity. Alternatively, the first intraoral image may be data obtained by scanning a plaster cast with a table scanner. The processor 310 may obtain the three-dimensional scan model by three-dimensionally modeling two-dimensional scan data received from the three-dimensional scanner 110. Specifically, the first intraoral image may be a three-dimensional scan model into which additional information is not inserted.

In an embodiment, the processor 310 may embed additional information, which is input through the user inputter 330, into the first intraoral image. Hereinafter, an image generated by embedding the additional information into the first intraoral image is referred to as a 'second intraoral image.'

To this end, the processor 310 may identify an area of the first intraoral image where the additional information is to be input. The processor 310 may automatically or manually identify an area of the first intraoral image where the additional information is to be input. Hereinafter, the area where the additional information is to be input is referred to as an additional information input area.

The first intraoral image is an image generated by scanning the oral cavity of the object, for example, the patient, and therefore includes intraoral information such as teeth and gingiva. Accordingly, the processor 310 may identify, as the additional information input area, a position of the first intraoral image that does not cover the teeth or the gingiva. In an embodiment, the processor 310 may automatically recognize an area of the first intraoral image including at least one of the teeth and the gingiva. The processor 310 may mask an area including at least one of the teeth and the gingiva in the first intraoral image and identify the additional information input area in the remaining area excluding the masked area.

In an embodiment, the processor 310 may receive a selection of at least one of the tooth and gingiva as a target through the user inputter 330. For example, when an upper right front tooth area among the patient's teeth is an important area to be treated, the user may select an upper right front tooth as the target tooth by using the user inputter 330. The processor 310 may identify the additional information input area in an area that does not cover the tooth or gingiva selected as the target. In the above example, the processor 310 may mask an area within a certain range from the upper right front tooth selected by the user and identify the additional information input area in the remaining area excluding the masked area.

In an embodiment, the processor 310 may identify the additional information input area from the first intraoral image by using a neural network, that is, a neural network trained to identify the additional information input area from a plurality of intraoral images. The neural network used in the disclosed embodiment may be a deep neural network (DNN). Additionally, the DNN may be formed as a convolution neural network (CNN). The convolutional neural network refers to a neural network that performs operations based on convolutional operations. The convolutional neural network may be a single neural network that comprehensively performs convolutional operations and other types of operations, or a neural network in which a plurality of neural networks are combined.

In the disclosed embodiment, the neural network may be a neural network implemented as a data recognition model and trained by using training data. The training data may be intraoral images. The neural network may use a trained data recognition model to distinguish between a specific area included in input data, for example, the tooth or gingival area, and other areas. The input image may be an image of the intraoral cavity of the patient to be treated. The neural network may receive an input image, perform a convolution operation to extract the teeth or gingiva, and generate an output image that identifies the additional information input area in the remaining areas excluding the tooth or gingiva area.

For example, the neural network may be included or stored in the processor 310. In this case, when the processor 310 inputs an intraoral image to the internal neural network, the neural network may output a result of identifying the additional information input area.

Alternatively, the neural network may be included in an external device (e.g., an external server, a computing device, etc.) separate from the intraoral image processing device 300. In this case, the processor 310 may transmit the intraoral image to the external device. The external device may input the received intraoral image to the neural network included within the external device. The neural network may be able to output the result of identifying the additional information input area. Subsequently, the external device may transmit the output result of the neural network to the intraoral image processing device 300. Accordingly, the processor 310 may identify the additional information input area based on the output result received from the external device.

In an embodiment, the processor 310 may embed additional information, which is input through the user inputter 330, into the first intraoral image.

The first intraoral image is a three-dimensional intraoral model, and the additional information input through the user inputter 330 may be two-dimensional data. In this case, the data format or extension of the first intraoral image may not match the data format or extension of the additional information. In an embodiment, the processor 310 may perform texture mapping so as to embed the additional information into the three-dimensional intraoral model. The texture mapping may refer to a method of mapping pixels of two-dimensional data to the surface of three-dimensional data.

In an embodiment, the processor 310 may embed the additional information into the three-dimensional scan data by performing the texture mapping and adjusting variables or color values of two-dimensional data pixels mapped to three-dimensional scan data.

In another embodiment, the processor 310 may embed the additional information into the three-dimensional scan data by changing or assigning colors of points or vertices of the three-dimensional scan data. Because the first intraoral image is three-dimensional scan data, the first intraoral image may be expressed as dots or mesh. In an embodiment, in order to embed the additional information into the three-dimensional scan data expressed as dots, the processor 310 may change, to another color, the color of the dot in the first intraoral image at a position corresponding to the outline of the text and image included in the additional information. Similarly, when the three-dimensional scan data is expressed as a mesh, the processor 310 may embed the additional information into the three-dimensional scan data by changing, to another color, the color of the vertex included in the mesh or a polygon including the vertex.

As described above, according to an embodiment, the intraoral image processing device 300 may identify the additional information input area.

Additionally, according to an embodiment, the intraoral image processing device 300 may obtain a second intraoral image by embedding the additional information, which is input to the additional information input area, into three-dimensional scan data.

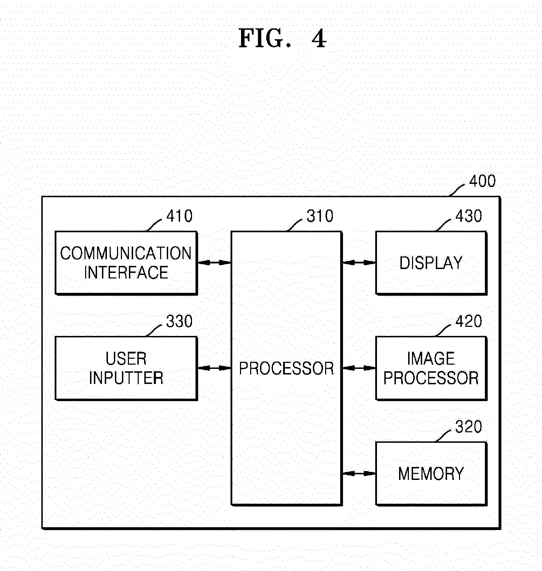

FIG. 4 is an internal block diagram of an intraoral image processing device according to an embodiment.

The intraoral image processing device 400 of FIG. 4 may be an example of the intraoral image processing device 300 of FIG. 3.

Referring to FIG. 4, the intraoral image processing device 400 may further include a communication interface 410, an image processor 420, and a display 430, in addition to the processor 310, the memory 320, and the user inputter 330.

Because the processor 310, the memory 320, and the user inputter 330 included in the intraoral image processing device 400 of FIG. 4 have the same functions of the processor 310, the memory 320, and the user inputter 330 included in the intraoral image processing device 300 of FIG. 3, respectively, the same reference numerals are used. Hereinafter, descriptions of parts redundant to those of the intraoral image processing device 300 of FIG. 3 are omitted.

The image processor 420 according to an embodiment may perform operations for generating and/or processing images. The image processor 420 may generate a first intraoral image based on raw data received from the three-dimensional scanner 110. The image processor 420 may generate a three-dimensional intraoral model by processing two-dimensional scan data.

In an embodiment, the image processor 420 may obtain a second intraoral image from the first intraoral image by performing texture mapping under the control of the processor 310. The image processor 420 may obtain the second intraoral image by embedding the additional information into the three-dimensional scan data by adjusting the variables or color values of the two-dimensional data pixels mapped to the three-dimensional scan data.

In another embodiment, the image processor 420 may obtain the second intraoral image by embedding the additional information into the three-dimensional scan data by changing the colors of points or vertices of the three-dimensional scan data under the control of the processor 310.

The display 430 according to an embodiment may output an intraoral image. The display 430 may output, on a screen, an intraoral image generated based on raw data received from the three-dimensional scanner 110. In addition, the display 430 may display a user interface screen including information related to a patient's dental treatment.

In an embodiment, the display 430 may output a user interface screen for selecting an additional information input area. The user interface screen for selecting the additional information input area may be a screen including the first intraoral image. The user may select a specific area of the first intraoral image as the additional information input area by using the user inputter 330 in response to the user interface screen for selecting the additional information input area. For example, the user may point to a specific area of the first intraoral image output on the interface screen by using a mouse, a keyboard, a touch panel, etc., and select the pointed area as the additional information input area. The processor 310 may identify the area selected by the user as the additional information input area.

In an embodiment, the display 430 may output a user interface screen on which the identified additional information input area is displayed. The processor 310 may output, at the point selected by the user, information indicating that the point is an area selected as the additional information input area. The processor 310 may display the position of the additional information input area to the user by using figures, arrows, lines, patterns, symbols, etc. In addition, the processor 310 may display the position of the additional information input area to the user by using a color different from an original color of the first intraoral image or a transparency different from an original transparency.

The user may input the additional information to the additional information input area by using the user inputter 330. The user may input desired texts as the additional information by using a keyboard, etc., or may input desired images as the additional information by using Paint, etc. In addition, the user may edit the additional information by using the user inputter 330. For example, the user may edit a font color or a font size of the additional information.

In an embodiment, the display 430 may output the additional information, which is input through the user inputter 330, to the additional information input area. The display 430 may output a user interface screen on which the additional information is displayed in the additional information input area.

In an embodiment, when the size of the additional information input through the user inputter 330 is outside the range of the additional information input area or is greater than or equal to a certain size, or when there are a plurality of additional information input areas in which the additional information is displayed, the display 430 may output an identifier indicating only the position of the additional information input area at the position of the additional information input area or may output only a portion of the additional information input area, instead of outputting all pieces of the input additional information on the screen.

The communication interface 410 according to an embodiment may perform communication with at least one external electronic device via a wired or wireless communication network. Specifically, the communication interface 410 may communicate with the three-dimensional scanner 110 under the control of the processor 310. In an embodiment, the communication interface 410 may receive two-dimensional image data from the three-dimensional scanner 110. In addition, the communication interface 410 may transmit control information to the three-dimensional scanner 110.

In an embodiment, the communication interface 410 may communicate with an external electronic device or a server connected via the wired or wireless communication network under the control of the processor 310. The external electronic device or the server may be a device or a server operated by a dental laboratory, etc. The communication interface 410 may transmit, to the server or the electronic device operated by the dental laboratory, etc., the first intraoral image generated based on image data obtained from the three-dimensional scanner 110. In addition, in an embodiment, the communication interface 410 may transmit, to the server or the electronic device operated by the dental laboratory, etc, the second intraoral image in which the additional information is embedded into the first intraoral image.

The communication interface 410 may include at least one short-range communication module that performs communication in accordance with a communication standard, such as Bluetooth, Wireless Fidelity (Wi-Fi), Bluetooth Low Energy (BLE), Near Field Communication/Radio Frequency Identification (NFC/RFID), Wi-Fi Direct, Ultra-Wideband (UWB), or ZigBee.

In addition, the communication interface 410 may further include a long-range communication module that communicates with a server for supporting long-range communication in accordance with a long-range communication standard. Specifically, the communication interface 410 may include a long-range communication module that performs communication via an Internet communication network. For example, the communication interface 410 may include a long-range communication module that performs communication via a communication network compliant with a communication standard, such as 3^{rd} generation (3G), 4^{th} generation (4G), and/or 5^{th} generation (5G).

In addition, the communication interface 410 may perform wired communication with the third-dimensional scanner 110 or the external electronic device operated by the dental laboratory, etc. To this end, the communication interface 410 may include at least one port for connecting to the third-dimensional scanner 110 or the external electronic device through a wired cable. The communication interface 410 may communicate with the third-dimensional scanner 110 or the external electronic device connected through at least one port by wire.

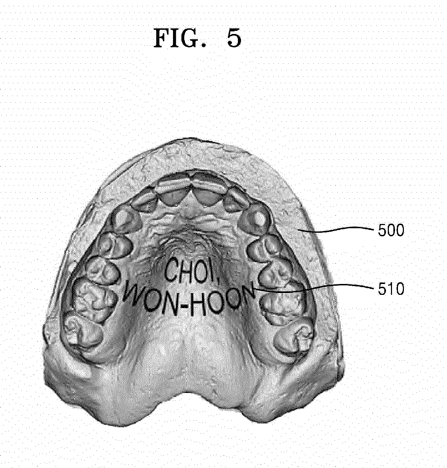

FIG. 5 is a diagram illustrating an obtained second intraoral image, according to an embodiment.

Referring to FIG. 5, the second intraoral image 500 may be an intraoral image representing a patient's upper jaw three-dimensionally. As illustrated in FIG. 5, the second intraoral image 500 may include embedded additional information 510. In FIG. 5, a case where the additional information 510 is information about a name of a patient receiving dental treatment is illustrated as an example.

The second intraoral image 500 may be obtained from a first intraoral image. The intraoral image processing device 400 may obtain a first intraoral image by three-dimensionally modeling raw data generated by scanning the patient's upper jaw.

The first intraoral image may be three-dimensional scan data. The third-dimensional scan data may have a certain format. For example, the third-dimensional scan data may have a format, such as STL, OBJ, and PLY. However, this is only one embodiment, and the data format of the first intraoral image is not limited thereto.

The intraoral image processing device 400 may receive additional information through the user inputter 330. The additional information input through the user inputter 330 may be a file having a format that is different from a format of the third-dimensional scan data. For example, the additional information may be a file having an extension, such as doc, hwp, jpg, pdf, or txt.

In an embodiment, the intraoral image processing device 400 may obtain the second intraoral image 500 by performing texture mapping to embed the additional information into the first intraoral image. Source data mapped to the first intraoral image, which is the third-dimensional scan data, may be a two-dimensional image. The texture mapping is a modeling method of projecting a two-dimensional image onto a surface of a three-dimensional model by mapping a position of a certain pixel on a two-dimensional image, that is, a pixel with U-axis and V-axis coordinate values (u, v) on a two-dimensional image to X-axis, Y-axis, and Z-axis coordinate values (x, y, z) on a third-dimensional image.

The intraoral image processing device 400 may adjust, to different values, the colors or other variables, i.e., parameter values, of pixels at positions corresponding to the additional information in the source data mapped to the third-dimensional scan data, that is, the second-dimensional image. The intraoral image processing device 400 may embed the additional information into the three-dimensional scan data by mapping, to the third-dimensional scan data, the two-dimensional image in which the colors or parameter values of the pixels are adjusted.

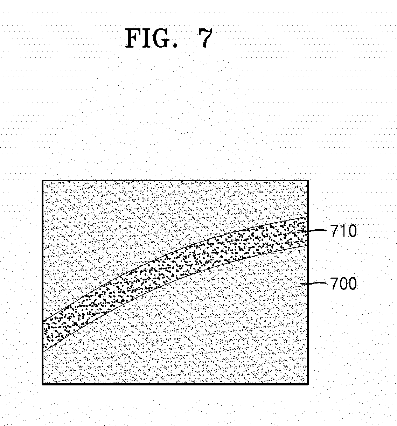

In another embodiment, the intraoral image processing device 400 may embed the additional information into the third-dimensional scan data by changing the colors of points or vertices of the third-dimensional scan data. This is described in detail below with reference to FIGS. 7 to 9.

Referring to FIG. 5, it may be seen that the additional information 510 included in the second intraoral image 500 is embedded to fit the third-dimensional scan data. That is, the second intraoral image 500 may be third-dimensional scan data including the embedded additional information 510. The intraoral image processing device 400 may transmit the second intraoral image 500 to the external electronic device or the server.

As described above, according to an embodiment, when it is desired to transmit the additional information to the external electronic device or the server, the intraoral image processing device 400 may transmit only the second intraoral image 500 without the need to generate and transmit a file or document separately from the from scan data. The external device or the server operated by the dental laboratory, etc. may use not only the image of the oral cavity but also the embedded additional information 510 from the second intraoral image 500, received from the intraoral image processing device 400.

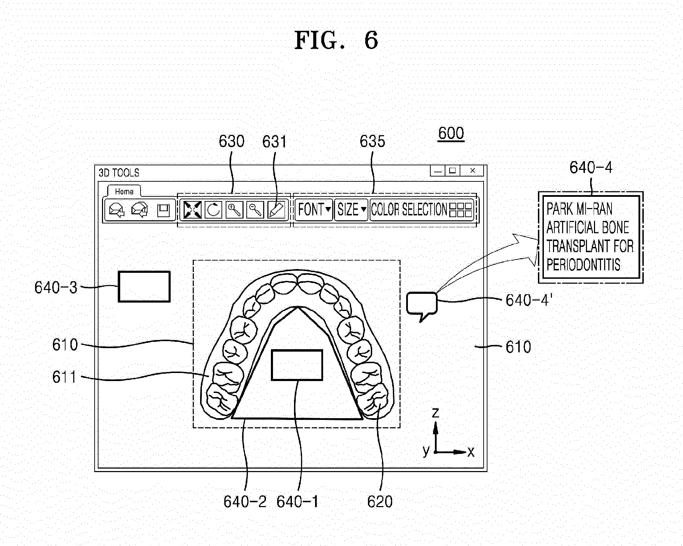

FIG. 6 is a diagram illustrating a user interface screen including an additional information input area, according to an embodiment.

In an embodiment, the intraoral image processing device 400 may obtain an intraoral image 610 representing a patient's upper jaw three-dimensionally. The intraoral image processing device 400 may generate a user interface screen 600 including the intraoral image 610 and visually output the user interface screen 600 on the display 430.

The user interface screen 600 may further include a menu bar 630 including at least one menu for editing or changing the intraoral image 610. For example, the menu bar 630 may include menus including zoom-in, zoom-out, full screen viewing, previous image viewing, angle or position change, etc. of the intraoral image 610.

In an embodiment, the menu bar 630 may further include a menu 631 for inputting additional information. In order to input additional information, the user may select, from the menu bar 630, the menu 631 for inputting additional information. When the menu 631 for inputting additional information is selected by the user, the intraoral image processing device 400 may output, on the user interface screen 600, an additional information input area 640 for inputting additional information.

To this end, first, the intraoral image processing device 400 may identify the additional information input area. The additional information input area may refer to an area where the additional information is to be input.

In an embodiment, the intraoral image processing device 400 may automatically recognize an oral cavity area 611 on the user interface screen 600. The oral cavity area 611 may refer to an area including at least one of teeth and gingiva. The intraoral image processing device 400 may mask the oral cavity area 611 among the areas included in the user interface screen 600 and identify the additional information input area in the remaining areas excluding the masked area.

Alternatively, the intraoral image processing device 400 may allow the user to select the teeth or gingiva to be treated. In this case, the intraoral image processing device 400 may identify the additional information input area in an area that is outside a certain range from the teeth or gingiva selected by the user.

In an embodiment, the intraoral image processing device 400 may identify the additional information input area from the user interface screen 600 by using a neural network trained to identify the additional information input area from a plurality of intraoral images.

In an embodiment, the intraoral image processing device 400 may output the identified additional information input area on the user interface screen 600. The intraoral image processing device 400 may display the additional information input area by using figures, arrows, lines, dots, patterns, symbols, color different from a surrounding color, transparency different from a surrounding transparency, a blinking cursor, etc.

For example, as illustrated in FIG. 6, the intraoral image processing device 400 may identify, as the additional information input area, an area located inside the oral cavity area 611 among areas other than the oral cavity area 611. The intraoral image processing device 400 may identify a random position inside the oral cavity area 611 as a first additional information input area 640-1 and may output the identified area on the user interface screen 600 in a square shape.

Alternatively, the intraoral image processing device 400 may identify the entire area inside the boundary of the oral cavity area 611 as a second additional information input area 640-2 and may output the identified area on the user interface screen 600 in a polygonal shape.

Alternatively, the intraoral image processing device 400 may identify a random position outside the oral cavity area 611 among areas other than the oral cavity area 611 as a third additional information input area 640-3 or a fourth additional information input area 640-4 and may output the identified additional information input area 640-3 or 640-4 on the user interface screen 600.

In another embodiment, the intraoral image processing device 400 may allow the user to directly select the additional information input area. For example, when the user clicks or touches a specific position on the user interface screen 600 by using a mouse, a keyboard, a touch panel, etc., the intraoral image processing device 400 may identify the specific position selected by the user as the additional information input area and may display the additional information input area in the identified area. When the area selected by the user is the oral cavity area 611 or an area adjacent to the oral cavity area 611, or when the area is within a certain range of the tooth or gingiva to be treated, the intraoral image processing device 400 may display that the relevant area is unable to be selected as the additional information input area, may generate the additional information input area in another area adjacent to the relevant area, or may not generate the additional information input area in the relevant area without separate indication.

In an embodiment, the intraoral image processing device 400 may receive the additional information from the user and may display the received additional information in the additional information input area 640 output on the current user interface screen 600. For example, when the first additional information input area 640-1 is displayed on the current user interface screen 600, the intraoral image processing device 400 may display and output the additional information received from the user in the first additional information input area 640-1. In this case, the additional information displayed in the first additional information input area 640-1 may be expressed according to the flexure of the intraoral image 610, or may be expressed in intaglio or embossing.

The user may input the additional information by using a program, a tool, or an application for inputting additional information, which is provided by the intraoral image processing device 400, and may edit the additional information. The additional information may have various forms, such as linguistic characters, special characters, numbers, punctuation marks, figures, arrows, lines, patterns, and symbols.

The intraoral image processing device 400 may further include a menu 635 for inputting and editing additional information on the user interface screen 600 so as to allow the user to input and edit the additional information in various forms. The menu 635 for inputting and editing additional information may include various menus for adjusting the type of additional information, font size, color, transparency, etc., or selecting special characters or symbols, but is not limited thereto. The user may select the menu 635 for inputting and editing additional information on the user interface screen 600 and may input additional information in a desired type of font and a desired size.

In an embodiment, when the input additional information is greater than a certain size, or when there are a plurality of additional information input areas where additional information is displayed, the intraoral image processing device 400 may not output all of the input additional information on the screen. Instead, the intraoral image processing device 400 may output the additional information input area whose size is reduced, may output only a portion of the additional information input area, or may output only an identifier indicating the position of the additional information input area on the user interface screen 600. The identifier indicating the position of the additional information input area may include, for example, speech bubbles, emoticons, icons, etc.

For example, in FIG. 6, when the additional information input to the fourth additional information input area 640-4 is long and the size of the fourth additional information input area 640-4 also increases depending on the length of the additional information, and thus, there is a risk of covering the oral cavity area 611, the intraoral image processing device 400 may output only a speech bubble-shaped identifier 640-4' indicating the position of the fourth additional information input area 640-4 on the user interface screen 600, instead of outputting the entire fourth additional information input area 640-4 on the screen, Thereafter, when the user clicks the identifier 640-4' or moves the cursor on the identifier 640-4' by using the mouse or the keyboard, the intraoral image processing device 400 may output all of the additional information input to the fourth additional information input area 640-4 on the user interface screen 600.

As described above, according to an embodiment, the intraoral image processing device 400 may automatically or manually identify the additional information input area. In addition, the intraoral image processing device 400 may receive additional information in various forms. In addition, the intraoral image processing device 400 may output a user interface screen on which the additional information input area is displayed in the identified additional information input area. In addition, the intraoral image processing device 400 may output a user interface screen on which the additional information input by the user is displayed in the additional information input area.

FIG. 7 is a diagram for describing a method of embedding additional information into an intraoral image, according to an embodiment.

In an embodiment, the intraoral image processing device 400 may obtain a second intraoral image 700 by embedding additional information 710 into the first intraoral image. Both the first intraoral image and the second intraoral image may be three-dimensional scan data. The three-dimensional scan data may be expressed as point cloud data, or may be expressed as mesh data generated through a technique such as triangulation with reference to point cloud data.

FIG. 7 is an enlarged view of the second intraoral image 700 and illustrates a case where the second intraoral image is expressed as numerous point groups.

The additional information may include texts, numbers, images, etc. For example, when the additional information includes texts, one independent letter may be formed by gathering the simplest basic structures forming the letter. The basic structures may include lines, points, etc.

In an embodiment, the intraoral image processing device 400 may identify the basic structure forming texts, numbers, images, etc. included in the additional information 710 in order to embed the additional information 710 into the first intraoral image. The intraoral image processing device 400 may change, to other colors, the colors of dots on the first intraoral image at positions corresponding to the basic structures such as lines or dots. in other words, the intraoral image processing device 400 may obtain the second intraoral image 700 displayed with the additional information 710 embedded into the scan data by changing, to other colors different from original colors, the colors of the dots on the three-dimensional scan data at the positions where the additional information 710 is to be embedded.

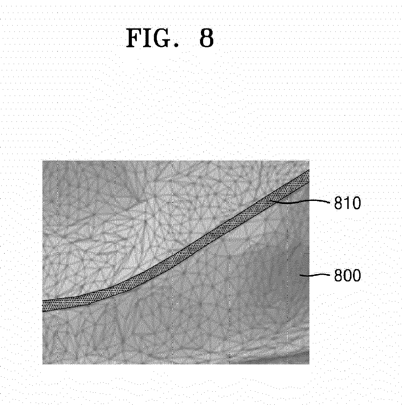

FIG. 8 is a diagram for describing a method of embedding additional information into an intraoral image, according to another embodiment.

In an embodiment, the intraoral image processing device 400 may obtain a second intraoral image 800 by embedding additional information 810 into a first intraoral image.

FIG. 8 is an enlarged view of the second intraoral image 800. The second intraoral image 800 may be three-dimensional scan data including numerous meshes. The mesh may include polygons of minimum unit, which are called a polygon. The polygon may be, for example, a triangle.

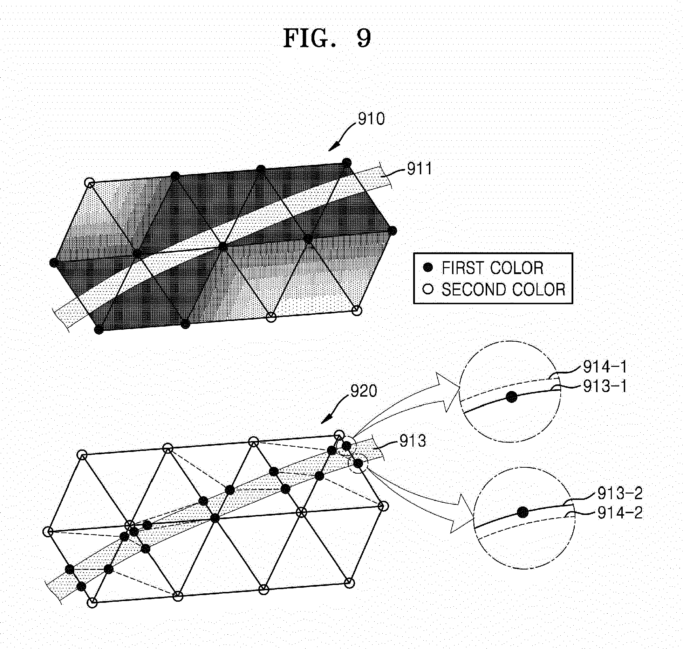

In an embodiment, the intraoral image processing device 400 may identify the basic structure forming the texts or images included in the additional information 810, and may embed the additional information 810 into the scan data by changing the color of the mesh on the first intraoral image at a position corresponding to the basic structure. A method of embedding the additional information 810 into the scan data by changing the color of the mesh is described below in more detail with reference to FIG. 9.

FIG. 9 is a diagram for describing embedding the additional information into the scan data by changing the color of the mesh, according to an embodiment.

FIG. 9 is a further enlarged view of the second intraoral image 800 of FIG. 8.

FIG. 9 illustrates second intraoral images 910 and 920 into which additional information 911 and 913 is embedded, and illustrates a case where the second intraoral images 910 and 920 are three-dimensional scan data including meshes. Each apex of the triangles forming the mesh may be referred to as a vertex. Each vertex has its own color. When two connected vertices have different colors, the triangle between the two vertices may be expressed as a color in which the two different colors are linearly interpolated and/or gradient. The method of expressing the case where the two connected vertices have different colors is not limited thereto. In addition, when three connected vertices of the triangle have the same color, the color of the surface of the triangle may be expressed in the same color as the color of the three vertices.

In an embodiment, the intraoral image processing device 400 may embed the additional information 911 and 913 into the first intraoral image by changing the colors of the vertices in the first intraoral image located adjacent to the positions where the additional information 911 and 913 passes or assigning colors to the vertices in the first intraoral image. When the line forming the additional information 911 and 913 has a certain thickness, the vertex adjacent to the positions where the additional information 911 and 913 passes may refer to the vertex located on both sides of the line having the certain thickness and closest to the line forming the additional information 911 and 913.

As illustrated in the upper diagram of FIG. 9, when the colors of the vertices in the first intraoral image located adjacent to the point where the additional information 911 passes are changed to a first color, or when a first color is assigned to the vertices in the first intraoral image, the triangles including the same vertices have the same color. That is, as illustrated in the upper drawing of FIG. 9, all triangles including the vertices adjacent to the positions where the additional information 911 passes have the first color, and thus, may be distinguished from other areas. However, the triangle that includes both the vertex having the first color and the vertex having the second color is expressed as a color in which the first color and the second color are linearly interpolated and/or gradient. The method of embedding the additional information 911 in the manner illustrated in the upper drawing of FIG. 9 is a method in which the additional information 911 is embedded more accurately at the corresponding position as the density of the mesh increases. On the other hand, when the density of the mesh is low, the degree of matching between the position where the additional information 911 passes and the color change of the vertices included in the mesh of the first intraoral image may be reduced.

In another embodiment, the intraoral image processing device 400 may embed the additional information 911 and 913 into the first intraoral image by changing the colors of the vertices in the first intraoral image at the positions where the additional information 911 and 913 passes or assigning colors to the vertices in the first intraoral image. That is, the intraoral image processing device 400 may embed the additional information 911 and 913 into the first intraoral image by changing, to colors different from original colors, the colors of the vertices included in the mesh through which the line forming the additional information 911 and 913 passes or assigning colors different from original colors to the vertices included in the mesh.

The lower diagram of FIG. 9 illustrates a method of increasing the number of triangles by splitting triangles in the first intraoral image located at positions corresponding to the position through which the additional information 913 passes. Referring to the lower drawing of FIG. 9, the intraoral image processing device 400 may add new vertices to the positions where the additional information 913 meets the mesh in the first intraoral image located at the position corresponding to the point where the additional information 913 passes. That is, the intraoral image processing device 400 may add vertices at positions where boundaries (edges) at which surrounding triangles and borderlines on both sides of the additional information 913 having a certain thickness are in contact with each other cross each other. In this case, the number of triangles including the vertices also increases. The intraoral image processing device 400 may distinguish between the triangles and other areas by changing, to the first color, the colors of the vertices in the first intraoral image at the positions where the additional information 913 passes or assigning the first color to the vertices in the first intraoral image so that the triangles including the same vertices have the same color. According to the lower drawing of FIG. 9, the number of triangles including the same vertices is greater than that in the upper drawing of FIG. 9, making it possible to more accurately embed the additional information 913 at the corresponding position in the first intraoral image.

However, even in the lower drawing of FIG. 9, as in the upper drawing of FIG. 9, the triangle that includes the vertex having the first color and the vertex having the second color is expressed as a color in which the first color and the second color are linearly interpolated and/or gradient. In an embodiment, when the line forming the additional information 913 has a certain thickness, the intraoral image processing device 400 may additionally form offset lines 914-1 and 914-2 at positions close to the borderlines 913-1 and 913-2 on both sides of the line. In addition, new vertices may be added at the points where the offset lines 914-1 and 914-2 meet the mesh. In this case, additional triangles are formed between the borderlines 913-1 and 913-2 on both sides of the line forming the additional information 913 and the offset lines 914-1 and 914-2. As the gap between the borderlines 913-1 and 913-2 on both sides of the line forming the additional information 913 and the offset lines 914-1 and 914-2 narrows, the area of triangles expressed in gradient colors is reduced, and thus, the additional information 913 may be more accurately embedded into the three-dimensional scan data.

However, the above-described method is only an embodiment, and the intraoral image processing device 400 may obtain the second intraoral image by embedding the additional information into the first intraoral image by using various other methods.

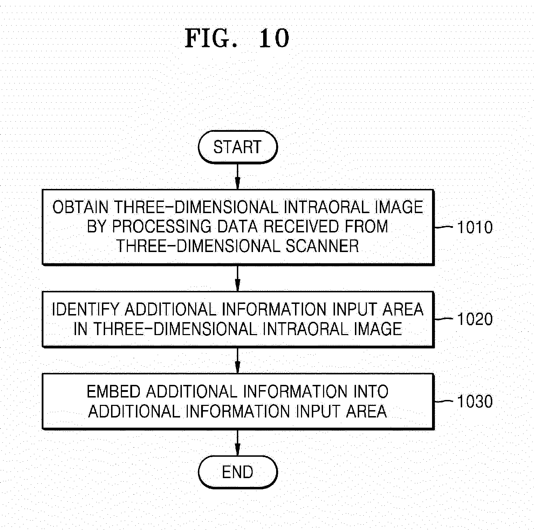

FIG. 10 is a flowchart illustrating a method of embedding additional information into an intraoral image, according to an embodiment.

Referring to FIG. 10, an intraoral image processing device may obtain a three-dimensional intraoral image by processing raw data received from a three-dimensional scanner (operation 1010).

The intraoral image processing device may identify an additional information input area in the three-dimensional intraoral image (operation 1020).

The intraoral image processing device may directly receive the additional information input area from a user or may automatically identify the additional information input area.

The intraoral image processing device may mask an intraoral area in the three-dimensional intraoral image and identify the additional information input area in the remaining areas other than the intraoral area.

Alternatively, the intraoral image processing device may identify the additional information input area in an area that is outside a certain range from a tooth or gingiva selected by the user as a treatment target.

The intraoral image processing device may receive the additional information from the user.

The intraoral image processing device may embed the additional information, which is input from the user, into the additional information input area (operation 1030).

The intraoral image processing device may embed the additional information into the intraoral image by replacing variables or color values of pixels of a two-dimensional image mapped to the intraoral image with values corresponding to the additional information. Alternatively, the intraoral image processing device may embed the additional information into the intraoral image by changing the color of at least one of the points or vertices of the intraoral image, which is located at a position corresponding to the outline of at least one of text and an image included in the additional information, and a triangle including the vertices,

The intraoral image processing method according to an embodiment of the present disclosure may be implemented in the form of program commands that are executable through a variety of computer means and may be recorded on a computer-readable recording medium. In addition, an embodiment of the present disclosure may be a computer-readable storage medium having recorded thereon one or more programs including at least one instruction for executing the intraoral image processing method.

In addition, the intraoral image processing method according to an embodiment of the present disclosure may be implemented as a computer program product including a computer-readable recording medium having recorded thereon a program for implementing the intraoral image processing method performed by the intraoral image processing device, the intraoral image processing method including obtaining a first intraoral image, and obtaining a second intraoral image, into which the input additional information is embedded, by embedding the input additional information into at least a partial area of the first intraoral image.

The computer-readable storage medium may include program commands, data files, data structures, etc. alone or in combination. Examples of the computer-readable storage medium may include magnetic media, such as hard disk, floppy disk, and magnetic tape, optical media, such as compact disc read-only memory (CD-ROM) and digital versatile disc (DVD), magneto-optical media, such as floptical disk, and hardware devices specially configured to store and execute program commands, such as read-only memory (ROM), random access memory (RAM), and flash memory.

A machine-readable storage medium may be provided in the form of a non-transitory storage medium. The 'non-transitory storage medium' may refer to a tangible device. For example, the 'non-transitory storage medium' may include a buffer in which data is temporarily stored.

According to an embodiment, the intraoral image processing methods according to various embodiments of the present disclosure may be provided by being included in a computer program product. The computer program product may be distributed in the form of a machine-readable storage medium (e.g., CD-ROM). Alternatively, the computer program product may be distributed (e.g., downloaded or uploaded) online, either via an application store (e.g., Play Store^{™}, etc.) or directly between two user devices (e.g., smartphones). Specifically, the computer program product according to the disclosed embodiment may include a storage medium having recorded thereon a program including at least one instruction for performing the intraoral image processing method according to the disclosed embodiment.

Although the embodiments have been described in detail, the scope of the present disclosure is not limited thereto, and various modifications and improvements made by using the basic concept of the present disclosure defined in the appended claims by those of ordinary skill in the art also fall within the scope of the present disclosure.

## Claims

1. An intra image processing method comprising:
obtaining a first intraoral image; and
obtaining a second intraoral image, into which input additional information is embedded, by embedding the input additional information into at least a partial area of the first intraoral image.

2. The intra image processing method of claim 1, further comprising identifying an additional information input area for displaying the additional information in the first intraoral image.

3. The intra image processing method of claim 2, wherein the identifying of the additional information input area comprises identifying the additional information input area in remaining areas other than an oral cavity area of the first intraoral image.

4. The intra image processing method of claim 2, further comprising receiving a selection of at least one of a tooth and gingiva as a target, wherein the identifying of the additional information input area comprises identifying the additional information input area in remaining area other than an area within a certain range from the at least one selected from the tooth and the gingiva.

5. The intra image processing method of claim 2, wherein the identifying of the additional information input area comprises identifying the additional information input area from the first intraoral image by using a neural network trained to identify additional information input areas from a plurality of intraoral images.

6. The intra image processing method of claim 2, further comprising
outputting a user interface screen for selecting the additional information input area,
wherein the identifying of the additional information input area comprises identifying a selected area as the additional information input area in response to the user interface screen.

7. The intra image processing method of claim 2, further comprising outputting at least one of a user interface screen on which the identified additional information input area is displayed and a user interface screen on which the input additional information is displayed in the identified additional information input area.

8. The intra image processing method of claim 7, wherein,
when the input additional information is greater than or equal to a certain size and when there are a plurality of additional information input areas where the additional information is displayed, the outputting of the user interface screen on which the input additional information is displayed comprises outputting an identifier indicating a position of the additional information input area instead of the additional information, or outputting the additional information input area whose size is reduced.

9. The intra image processing method of claim 1, wherein the additional information comprises at least one of a text and an image.

10. The intra image processing method of claim 1, wherein
the first intraoral image is three-dimensional scan data, and
the obtaining of the second intraoral image into which the additional information is embedded comprises obtaining the second intraoral image by embedding the additional information into the first intraoral image by replacing variables or color values of pixels of a two-dimensional image mapped to the first intraoral image with values corresponding to the additional information.

11. The intra image processing method of claim 1, wherein
the first intraoral image is three-dimensional scan data expressed as at least one of dots and mesh, and
the obtaining of the second intraoral image into which the additional information is embedded comprises obtaining the second intraoral image by embedding the additional information in the first intraoral image by changing a color of at least one of a point, a vertex, and a polygon including the vertex of the first intraoral image located at a position corresponding to an outline of at least one of a text and an image included in the additional information,

12. An intraoral image processing device comprising
a processor configured to execute one or more instructions,
wherein the processor is configured to execute the one or more instructions to:
obtain a first intraoral image; and
obtain a second intraoral image, into which input additional information is embedded, by embedding the input additional information into at least a partial area of the first intraoral image.

13. A computer-readable recording medium having recorded thereon a program for implementing an intraoral image processing method, wherein the intraoral image processing method comprises:
obtaining a first intraoral image; and
obtaining a second intraoral image, into which input additional information is embedded, by embedding the input additional information into at least a partial area of the first intraoral image.
